# EUROPÄISCHE PATENTSCHRIFT

(11) **EP 2 873 430 B1**
(45) Veröffentlichungstag und Bekanntmachung des Hinweises auf die Patenterteilung: **20.03.2019**
(21) Anmeldenummer: 13005428.1
(22) Anmeldetag: 19.11.2013
(51) Int. Cl.: A61M 5/315, A61M 5/31

(54) **Griffelement für eine Spritze**
Handle element for a syringe
Manette pour une seringue

(43) Veröffentlichungstag der Anmeldung: 20.05.2015
(73) Patentinhaber: Gerresheimer Regensburg GmbH, 93047 Regensburg (DE)
(72) Erfinder: Pfrang, Jürgen, 93183 Kallmünz (DE)
(74) Vertreter: Hannke, Christian

(56) Entgegenhaltungen:
- EP-A2- 1 566 194
- DE-B4-102005 005 468
- US-A- 5 207 646
- US-A1- 2008 097 338
- US-A1- 2009 036 839

## Beschreibung

Die Erfindung betrifft ein Griffelement zur Befestigung an einen Spritzenzylinder einer Spritze, die eine Kolbenstange, an deren einem Ende sich ein Kolbenstopfen befindet, aufweist, wobei das Griffelement unmittelbar hinter dem vorderseitigen Ender der Kolbenstange, an dem der Kolbenstopfen befestigbar ist, angeordnet ist.

Die Verwendung von gattungsgemäßen Griffelementen im Bereich der Spritzen ist weit verbreitet. In der Patentschrift DE 102005005468B4 wird beispielsweise eine Spritze mit einem Griffelement offenbart, welche zweiteilig ausgebildet ist und eine Verriegelungsfunktion beinhaltet, die das Griffelement mit dem Spritzenzylinder mithilfe von Rastarmen und Rastnasen verbindet. In der Patentschrift US 2009036839A wird ein Griffelement, welches entgegen der Injektionsrichtung montiert wird und somit das Griffelement vergrößert, beschrieben.

Bei allen zuvor genannten Lösungen liegt das Griffelement als zusätzliches Bauteil vor. Dies erfordert neben dem zusätzlichen Werkzeug zum Herstellen des Bauteils auch zusätzliche Handhabungsschritte zum Montieren des Griffelements.

Bei sämtlichen aus dem Stand der Technik bekannten Griffelementen ist ein zusätzlicher Montageschritt erforderlich um das Griffelement an der Spritze zu befestigen, was für den Abfüller oder Anwender zusätzlichen Aufwand bedeutet. Bei den bekannten Herstellungsmethoden müssen die Teile, wie Kolbenstange und Griffelement, einzeln hergestellt werden, was zudem mehrere Prozessschritte erfordert. In gleicher Weise gilt dies für die Herstellung bzw. das Handling eines zusätzlichen Siegels.

Die Aufgabe der vorliegenden Erfindung besteht darin, ein Griffelement für Spritzen bereit zu stellen, welches zum einem die Auflagefläche vergrößert und zum anderen besonders einfach zu montieren, herzustellen und somit besonders kostengünstig zu realisieren ist.

Die Aufgabe der Erfindung wird durch ein Griffelement (6) zur Befestigung an einen Spritzenzylinder (2) einer Spritze (1), die eine Kolbenstange (3), an deren einem Ende sich ein Kolbenstopfen (11) befindet, wobei das Griffelement (6) unmittelbar hinter dem vorderseitigen Ende der Kolbenstange (3), an dem der Kolbenstopfen (11) befestigbar ist, angeordnet ist gelöst. Dieses Griffelement zeichnet sich dadurch aus, dass das Griffelement (6) und die Kolbenstange (3) vor der Befestigung am Spritzenzylinder durch einen oder mehrere Verbindungsstege verliersicher miteinander verbunden sind und das Griffelement (6) und die Kolbenstange (3) gemeinsam in einem Spritzgießverfahren hergestellt werden.

Das Griffelement und die Kolbenstange sind vor der Befestigung an dem Spritzenzylinder verliersicher miteinander verbunden. Insbesondere wird die Aufgabe der Erfindung dadurch gelöst, dass das Griffelement und die Kolbenstange in einem gemeinsamen Herstellverfahren gefertigt werden. Durch die gemeinsame Fertigung werden Prozessschritte verringert, indem die Befestigung des Griffelements an den Spritzenkolben nun im gleichen Schritt wie die Montage der Kolbenstange in den Spritzenzylindern erfolgt.

Zur Anwendung der vorliegenden Erfindung eignen sich unterschiedliche Arten von Spritzen. Sie findet Anwendung bei vorgefüllten und nicht vorgefüllten Spritzen, außerdem kann die Spritze aus einem beliebigen Material bestehen. Insbesondere bestehen derartige Spritzen beispielsweise aus Glas oder Kunststoff. Der Spritzenzylinder kann bereits mit einer Kanüle ausgestatten sein oder erst nachträglich, z.B. durch Einkleben, damit vereint werden.

Vorteilhafterweise werden im Sinne der vorliegenden Erfindung das Griffelement und die Kolbenstange im gleichen Spritzgießverfahren hergestellt. Dadurch wird der Vorteil der erleichterten Montage der Kolbenstange, des Griffelements und des Spritzenzylinders erreicht.

Besonders bevorzugt werden im Sinne der vorliegenden Erfindung das Griffelement und die Kolbenstange in einem Mehrkomponentenspritzgießverfahren gefertigt. Dadurch lassen sich verschiedene funktionsorientierte Werkstoffanforderungen für Kolbenstange und Griffelement realisieren.

In einer weiteren vorteilhaften Ausgestaltung werden im Sinne der vorliegenden Erfindung das Griffelement und die Kolbenstange in einem Montagespritzgießverfahren gefertigt. Dadurch kann die Verliersicherheit von Kolbenstange und Griffelement unabhängig von einer stoffschlüssigen Verbindung und damit in einer besonders einfachen Art und Weise erreicht werden.

Die Form des Griffelements ist vorteilhafterweise so ausgebildet, dass ein Anwender die Spritze bequem anwenden kann, ohne eine Gefahr einzugehen von dieser abzurutschen. Die Außenform ist daher frei wählbar, jedoch muss die Aussparung mittig des Griffelements den Abmessungen der Kolbenstange angepasst werden, so dass das Griffelement während der Injektion beweglich auf der Kolbenstange gehalten ist. Bevorzugter Weise sind die Abmessungen von Kolbenstopfen, Kolbenstange und Griffelement derart gewählt, dass auch nach der Befestigung von Kolbenstange und Griffelement am Spritzenzylinder die Kolbenstange nicht aus dem Griffelement herausziehbar ist.

Die Anordnung des Griffelements an der Kolbenstange erfolgt zwischen einer Auflagefläche der Kolbenstange und dem vorderseitigen Ende der Kolbenstange, an dem in der Regel ein Kolbenstopfen zur Abdichtung des Spritzenzylinders befestigbar ist. Vorteilhafterweise ist das Griffelement unmittelbar hinter dem vorderseitigen Ende der Kolbenstange angeordnet.

In einer vorteilhaften Ausführung der Erfindung bewirken die zur Befestigung von Kolbenstange und Griffelement auf den Spritzenzylinder notwendigen Kräfte eine Trennung der Kolbenstangeneinheit von der Griffelementeinheit an einer definierten Stelle.

Die Verbindung zwischen Kolbenstange und Griffelement kann beispielsweise über einen oder mehrere Verbindungsstege, die bei Befestigung als Sollbruchstellen fungieren, realisiert werden.

In einer weiteren vorteilhaften Ausführung der Erfindung ist das Griffelement an der Kolbenstange derartig angebracht, dass die Kolbenstange sich nach einer ersten Betätigung und der Überschreitung einer dafür definierten Betätigungskraft, von dem Griffelement an einer definierten Stelle trennt.

Die Verbindung zwischen Kolbenstange und Griffelement kann beispielsweise über einen oder mehrere Verbindungsstege, die bei Betätigung als Sollbruchstellen fungieren, realisiert werden.

In diesem Zusammenhang sei noch erwähnt, dass die definierte Stelle an der Kolbenstange sowohl durch Verbindungsstege als auch durch eine geringere Materialstärke gebildet sein kann. Die Verbindungsstege können im Sinne der vorliegenden Erfindung variabel ausgebildet sein. Es ist denkbar, die Verbindungsstege segmentartig oder mittels eines oder mehrerer Stege auszugestalten.

Die Befestigung des Griffelements am Spritzenzylinder erfolgt mittels einer Verrastung mit Hilfe von einem oder mehreren Rastelementen. Die Rastelemente befinden sich auf der dem Kolbenstopfen zugewandten Seite des Griffelements. Die Rastelemente überschnappen zur Funktionserfüllung den rückseitigen Kragen des Spritzenzylinders. Hierzu sind die Rastelemente vorteilhafterweise als biegsame Schnapphaken ausgebildet. Die Rastelemente können je nach Anforderungen und notweniger Geometrie in ihrer Ausgestaltung variieren, um eine geeignete Verbindung zwischen Spritzenzylinder und Griffelement herzustellen.

Vorteilhafterweise umfasst die Anordnung ein Griffelement nach einem der hier beschriebenen Merkmale. Weitere Vorteile, Ziele und Eigenschaften vorliegender Erfindung werden anhand anliegender Zeichnung und nachfolgender Beschreibung erläutert, in welchen beispielhaft ein Griffelement in Anordnung mit einer Spritze dargestellt und beschrieben ist.

In den Zeichnungen zeigen:
- Figur 1: schematisch eine Gesamtansicht einer Spritze mit Spritzenzylinder, Kolbenstange und Griffelement;
- Figur 2: schematisch einen Längsschnitt der Gesamtansicht einer Spritze mit Spritzenzylinder, Kolbenstange und Griffelement;
- Figur 3: schematisch eine längsgeschnittene Gesamtansicht einer Spritze mit Kolbenstange und Griffelement nach der Befestigung des Griffelements;
- Figur 4: schematisch eine längsgeschnittene Gesamtansicht einer Spritze mit Kolbenstange und Griffelement nach einer ersten Betätigung der Kolbenstange; und
- Figur 5: schematisch eine längsgeschnittene Detailansicht der Anordnung von Kolbenstange und Griffelement.

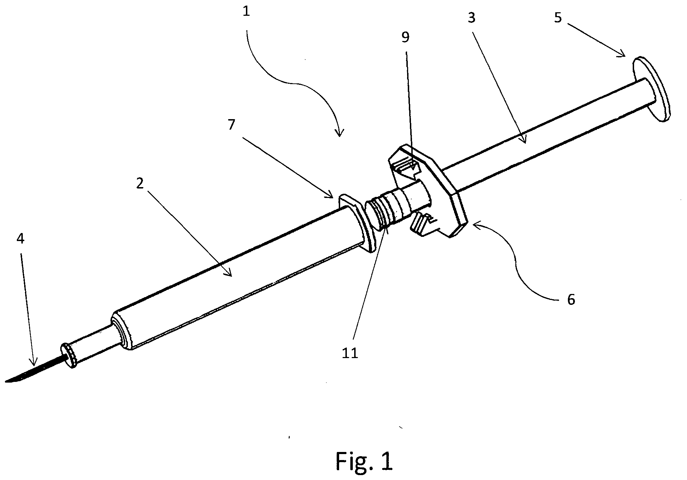

Die in den Fig. 1 bis 5 dargestellte Ausführungsform zeigt eine Anordnung aus einem Spritzenzylinder 2, einer Kolbenstange 3 und einem Griffelement 6 oder Teile dieser Anordnung.

Der Spritzenzylinder 2 umfasst eine Kanüle 4, die an einem Ende des Spritzenzylinders 2 montiert ist, und einen flanschartigen Kragen 7, der an der gegenüber der Kanüle 4 gelegenen Seite des Spritzenzylinders 2 ausgebildet ist.

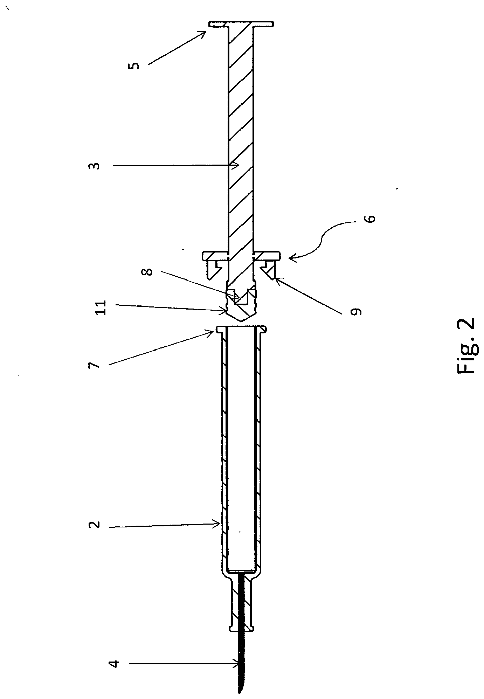

Die Kolbenstange 3 umfasst eine Auflagefläche 5, die am rückseitigen Ende der Kolbenstange 3 ausgebildet ist, eine Kolbenstangenschnittstelle 8 (siehe Fig. 2 bis 5), die an der gegenüber der Auflagefläche 5 gelegenen Seite ausgebildet ist, einen ersten Kolbenstangenabschnitt mit einem Durchmesser 15, der sich unmittelbar in der Nähe der Kolbenstangenschnittstelle 8 befindet und einen zweiten Kolbenstangenabschnitt mit einem Durchmesser 14. Bei der Montage wird ein Kolbenstopfen 11 an die Kolbenstangenschnittstelle 8 angebracht.

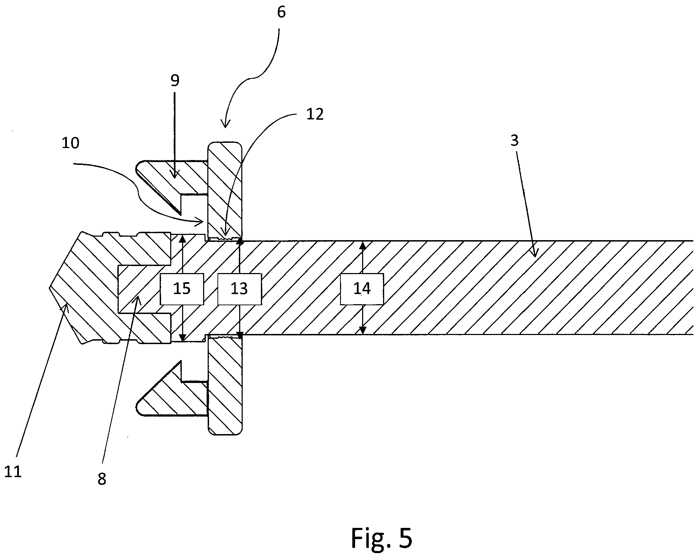

Das Griffelement 6 umfasst mehrere Rastelemente 9, die sich an der dem Kolbenstopfen zugewandten Seite 10 des Griffelements 6 befinden. Außerdem umfasst das Griffelement 6, wie in Fig. 5 ersichtlich, eine Aussparung mit einem Durchmesser 13.

Die Rastelemente 9 des Griffelements 6 sind als schnapphakenförmige Elemente ausgebildet und sind, wie der Fig. 1 zu entnehmen, so angeordnet, dass die Rastelemente 9 über den flanschartigen Kragen 7 überschnappen können.

Das Griffelement 6 ist durch Verbindungsstege 12 an der Kolbenstange 3 angebracht.

In den Fig. 1 und 2 wird eine Spritze 1 gezeigt, bei der die Kolbenstange 3 mit dem Griffelement 6 noch nicht an dem Spritzenzylinder 2 montiert sind.

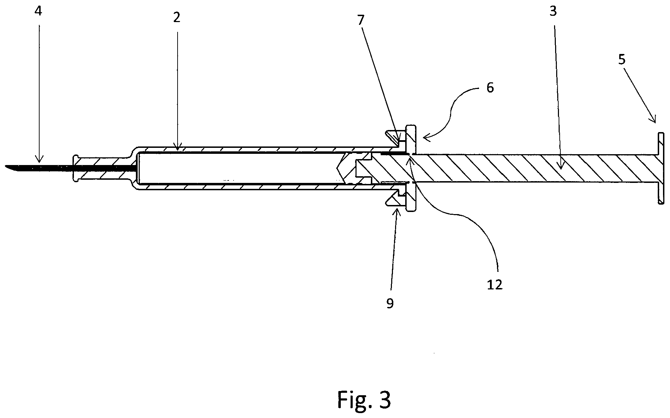

In der Fig. 3 wird eine Spritze 1 gezeigt, bei der das Griffelement 6 an den flanschartigen Kragen 7 des Spritzenzylinders 2 eingerastet ist. Die Kolbenstange 3 ist noch nicht von dem Griffelement 6 getrennt, sondern mit Hilfe der Verbindungsstege 12 mit dem Griffelement 6 verbunden.

Die Verrastung des Griffelements 6 mit dem flanschartigen Kragen 7 des Spritzenzylinders 2 erfolgt dadurch, dass beim Hineinschieben der Kolbenstange 3 in den Spritzenzylinder 2 die Rastelemente 9, die sich auf dem Griffelement 6 befinden, über den flanschartigen Kragen 7 überschnappen. Das Überschnappen ist durch die abgeschrägten schnapphakenförmigen Rastelemente 9 möglich.

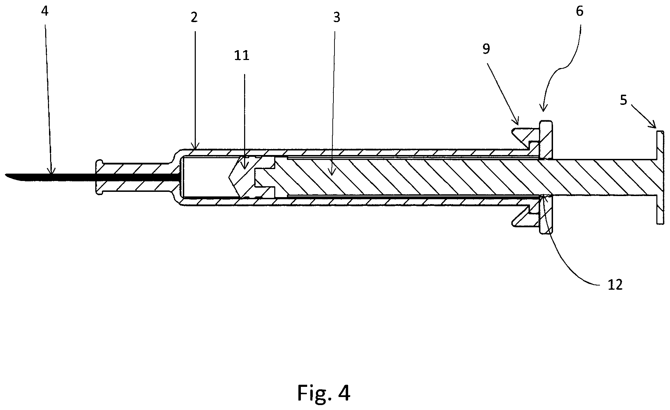

In der Fig. 4 wird eine Spritze 1 nach dem Trennen der Kolbenstange 3 von dem Griffelement 6 aufgezeigt. Dabei werden die Kolbenstange 3 und das Griffelement 6 an den Verbindungsstege 12 durch eine axiale Bewegung der Kolbenstange 3 getrennt.

Die Fig. 5 zeigt die Kolbenstange 3, an dessen einen Ende sich der Kolbenstopfen 11 befindet, und das von der Kolbenstange getrennte Griffelement 6. Durch die unterschiedliche Auslegung der Durchmesser 13, 14 und 15 ist es nicht möglich die Kolbenstange 3 aus dem Griffelement 6 herauszuziehen. Der Durchmesser 13 der Aussparung des Griffelements 6 ist kleiner ausgebildet als der Durchmesser 15 des ersten Kolbenstangenabschnitts, jedoch größer als der Durchmesser 14 des zweiten Kolbenstangenabschnitts.

Es versteht sich, dass es sich bei den vorstehend erläuterten Ausführungsbeispielen lediglich um eine bevorzugte Ausgestaltungen von erfindungsgemäßen Spritzen mit Griffelementen handelt. Insofern beschränkt sich die Ausgestaltung der Erfindung nicht auf dieses Ausführungsbeispiel.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Spritze
- 2: Spritzenzylinder
- 3: Kolbenstange
- 4: Kanüle
- 5: Auflagefläche
- 6: Griffelement
- 7: Flanschartiger Kragen
- 8: Kolbenstangenschnittstelle
- 9: Rastelemente
- 10: Dem Kolbenstopfen zugewandte Seite
- 11: Kolbenstopfen
- 12: Verbindungsstege
- 13: Durchmesser der Aussparung des Griffelements
- 14: Durchmesser eines zweiten Kolbenstangenabschnitts
- 15: Durchmesser eines ersten Kolbenstangenabschnitts

## Patentansprüche

1. Griffelement (6) zur Befestigung an einem Spritzenzylinder (2) einer Spritze (1), mit einer am Griffelement (6) angeordneten Kolbenstange (3), an deren einem Ende sich ein Kolbenstopfen (11) befindet, wobei das Griffelement (6) unmittelbar hinter dem vorderseitigen Ende der Kolbenstange (3), an dem der Kolbenstopfen (11) befestigbar ist, angeordnet ist,
**dadurch gekennzeichnet, dass**
das Griffelement (6) und die Kolbenstange (3) vor der Befestigung am Spritzenzylinder (2) durch einen oder mehrere Verbindungsstege (12) verliersicher miteinander verbunden sind, wobei die Kolbenstange (3) durch Überschreiten einer Betätigungskraft an den Verbindungsstegen (12) von dem Griffelement (6) trennbar ist und der Durchmesser (13) einer Aussparung des Griffelements (6) kleiner als der Durchmesser (15) eines ersten, distalen Kolbenstangenabschnitts und/oder des Durchmessers des Kolbenstopfens (11) und größer als der Durchmesser (14) eines zweiten, proximalen Kolbenstangenabschnitts ausgebildet ist und das Griffelement (6) und die Kolbenstange (3) gemeinsam in einem Spritzgießverfahren hergestellt worden sind.

2. Griffelement (6) nach Anspruch 1,
**dadurch gekennzeichnet, dass**
das Griffelement (6) und die Kolbenstange (3) in einem Mehrkomponentenspritzgießverfahren hergestellt werden.

3. Griffelement (6) nach Anspruch 1,
**dadurch gekennzeichnet, dass**
das Griffelement (6) und die Kolbenstange (3) in einem Montagespritzgießverfahren hergestellt werden.

4. Griffelement (6) nach einem der vorhergehenden Ansprüche,
**dadurch gekennzeichnet, dass**
das Griffelement (6) gemeinsam mit der Kolbenstange (3) an den Spritzenzylinder (2) montierbar ist.

5. Griffelement (6) nach Anspruch 4,
**dadurch gekennzeichnet, dass**
eine Trennung des Griffelements (6) von der Kolbenstange (3) durch eine erste Betätigung der Kolbenstange (3) nach der Befestigung erfolgt.

6. Griffelement (6) nach Anspruch 4,
**dadurch gekennzeichnet, dass**
eine Trennung des Griffelements (6) von der Kolbenstange (3) durch eine axialgerichtete Betätigung der Kolbenstange (3) erfolgt.

7. Griffelement (6) nach einem der vorhergehenden Ansprüche,
**dadurch gekennzeichnet, dass**
an der dem Kolbenstopfen zugewandten Seite (10) des Griffelements (6) mindestens ein Rastelement (9) angeordnet ist.

8. Griffelement (6) nach Anspruche 7,
**dadurch gekennzeichnet, dass**
das mindestens eine Rastelement (9) schnapphakenförmig ausgebildet ist.

9. Griffelement (6) nach Anspruch 7 oder 8,
**dadurch gekennzeichnet, dass**
das mindestens eine Rastelement (9) derart ausgebildet ist, dass es über einen Kragen (7) des Spritzenzylinders (2) überschnappen kann.

## Claims

1. Handle element (6) for fastening to a syringe cylinder (2) of a syringe (1), comprising a piston rod (3) arranged on the handle element (6), a piston stopper (11) being on one end thereof, the handle element (6) being arranged directly behind the front end of the piston rod (3), to which the piston stopper (11) can be fastened,
**characterised in that**
the handle element (6) and the piston rod (3) are captively interconnected by means of one or more connection webs (12) before being fastened to the syringe cylinder (2), it being possible to separate the piston rod (3) from the handle element (6) by exceeding an actuating force upon the connection webs (12), and the diameter (13) of a recess in the handle element (6) being smaller than the diameter (15) of a first, distal piston rod portion and/or the diameter of the piston stopper (11) and larger than the diameter (14) of a second, proximal piston rod portion, and the handle element (6) and the piston rod (3) having been produced together in an injection moulding method.

2. Handle element (6) according to claim 1,
**characterised in that**
the handle element (6) and the piston rod (3) are produced in a multi-component injection moulding method.

3. Handle element (6) according to claim 1,
**characterised in that**
the handle element (6) and the piston rod (3) are produced in an assembly injection moulding method.

4. Handle element (6) according to any of the preceding claims,
**characterised in that**
the handle element (6) together with the piston rod (3) is mountable on the syringe cylinder (2).

5. Handle element (6) according to claim 4,
**characterised in that**
the handle element (6) is separated from the piston rod (3) by means of a first actuation of the piston rod (3) after fastening.

6. Handle element (6) according to claim 4,
**characterised in that**
the handle element (6) is separated from the piston rod (3) by means of an axially directed actuation of the piston rod (3).

7. Handle element (6) according to any of the preceding claims,
**characterised in that**
at least one latching element (9) is arranged on the side (10) of the handle element (6) facing the piston stopper.

8. Handle element (6) according to claim 7,
**characterised in that**
the at least one latching element (9) is designed as a snap-in hook.

9. Handle element (6) according to either claim 7 or claim 8,
**characterised in that**
the at least one latching element (9) is designed such that it can snap over a collar (7) of the syringe cylinder (2).

## Revendications

1. Manette (6) pour la fixation sur un cylindre de seringue (2) d'une seringue (1), ayant une tige de piston (3) qui est disposée sur la manette (6) et à une extrémité de laquelle se trouve un bouchon de piston (11), la manette (6) étant disposé directement derrière l'extrémité côté avant de la tige de piston (3) sur laquelle le bouchon de piston (11) est apte à être fixé,
**caractérisé par le fait que**
la manette (6) et la tige de piston (3) sont reliés entre eux de façon imperdable par une ou plusieurs nervures de liaison (12) avant la fixation sur le cylindre de seringue (2), la tige de piston (3) étant apte à être séparée de la manette (6) par dépassement d'une force d'actionnement sur les nervures de liaison (12) et le diamètre (13) d'un évidement de la manette (6) étant formé plus petit que le diamètre (15) d'une première section de tige de piston, distale, et/ou que le diamètre du bouchon de piston (11) et plus grand que le diamètre (14) d'une seconde section de tige de piston, proximale, et la manette (6) et la tige de piston (3) ont été obtenus conjointement dans un procédé de moulage par injection.

2. Manette (6) selon la revendication 1,
**caractérisé par le fait que**
la manette (6) et la tige de piston (3) sont obtenus dans un procédé de moulage par injection à plusieurs composants.

3. Manette (6) selon la revendication 1,
**caractérisé par le fait que**
la manette (6) et la tige de piston (3) sont obtenus dans un procédé de moulage par injection en montage.

4. Manette (6) selon l'une des revendications précédentes,
**caractérisé par le fait que**
la manette (6) est apte à être monté conjointement avec la tige de piston (3) sur le cylindre de seringue (2).

5. Manette (6) selon la revendication 4,
**caractérisé par le fait que**
une séparation de la manette (6) de la tige de piston (3) a lieu après la fixation par un premier actionnement de la tige de piston (3).

6. Manette (6) selon la revendication 4,
**caractérisé par le fait que**
une séparation de la manette (6) de la tige de piston (3) a lieu par un actionnement dirigé axialement de la tige de piston (3).

7. Manette (6) selon l'une des revendications précédentes,
**caractérisé par le fait que**
au moins un élément d'encliquetage (9) est disposé sur le côté (10) de la manette (6) tourné vers le bouchon de piston.

8. Manette (6) selon la revendication 7,
**caractérisé par le fait que**
ledit au moins un élément d'encliquetage (9) est réalisé en forme de crochet d'encliquetage.

9. Manette (6) selon l'une des revendications 7 ou 8,
**caractérisé par le fait que**
ledit au moins un élément d'encliquetage (9) est formé de telle sorte qu'il peut s'encliqueter sur une collerette (7) du cylindre de seringue (2).
